# EUROPÄISCHE PATENTANMELDUNG

(11) **EP 0 916 661 A1**
(43) Veröffentlichungstag der Anmeldung: **19.05.1999**
(21) Anmeldenummer: 98119584.5
(22) Anmeldetag: 16.10.1998
(51) Int. Cl.: C07D 261/10

(54) **Verfahren zur Herstellung von 3,5-Dimethylisoxazol-4-sulfochlorid**

(30) Priorität: 29.10.1997 DE 19747625
(71) Anmelder: Bayer Aktiengesellschaft, 51368 Leverkusen (DE)
(72) Erfinder: Gallenkamp, Bernd Dr., 42113 Wuppertal (DE); Rohe, Lothar Dr., 42113 Wuppertal (DE)

(57) **Zusammenfassung**

Nach einem neuen Verfahren läßt sich 3,5-Dimethylisoxazol-4-sulfochlorid herstellen, indem man 3,5-Dimethylisoxazol zunächst mit Chlorsulfonsäure und danach zusätzlich mit Thionylchlorid bei Temperaturen zwischen 60°C und 110°C umsetzt.

## Beschreibung

Die vorliegende Erfindung betrifft ein neues Verfahren zur Herstellung des bekannten 3,5-Dimethylisoxazol-4-sulfochlorids, das als Zwischenprodukt zur Herstellung von Wirkstoffen mit mikrobiziden Eigenschaften verwendbar ist.

Es ist bereits bekannt geworden, daß sich 3,5-Dimethylisoxazol-4-sulfochlorid herstellen läßt, indem man 3,5-Dimethylisoxazol mit Chlorsulfonsäure umsetzt (vgl. J. Het. Chem. 18 (1981), 997-1006). Nachteilig an diesem Verfahren ist aber, daß das gewünschte Produkt nur in relativ niedriger Ausbeute anfällt.

Es wurde nun gefunden, daß man 3,5-Dimethylisoxazol-4-sulfochlorid der Formel 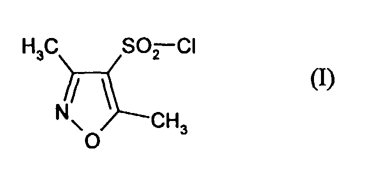 erhält, wenn man 3,5-Dimethylisoxazol der Formel 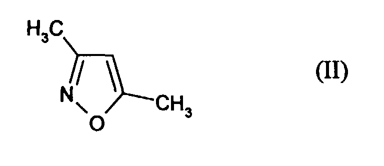 zunächst mit Chlorsulfonsäure und danach zusätzlich mit Thionylchlorid bei Temperaturen zwischen 60°C und 110°C umsetzt.

Es ist als ausgesprochen überraschend zu bezeichnen, daß sich 3,5-Dimethyl-isoxazol-4-sulfochlorid der Formel (I) nach dem erfindungsgemäßen Verfahren in wesentlich höherer Ausbeute herstellen laßt als nach der bisher bekannten Methode.

Das erfindungsgemäße Verfahren zeichnet sich durch eine Reihe von Vorteilen aus. So ermöglicht es, wie schon erwähnt, die Synthese von 3,5- Dimethylisoxazol-4-sulfochlorid der Formel (I) in sehr hoher Ausbeute. Günstig ist auch, daß die benötigten Reaktionskomponenten einfach herstellbar und auch in größeren Mengen verfügbar sind. Ein weiterer Vorteil besteht schließlich darin, daß die Durchführung der Umsetzung und die Isolierung des Reaktionsproduktes keinerlei Schwierigkeiten bereitet.

Setzt man 3,5-Dimethylisoxazol zunächst mit Chlorsulfonsäure um und fügt anschließend Thionylchlorid hinzu, so kann der Verlauf des erfindungsgemäßen Verfahrens durch das folgende Formelschema veranschaulicht werden.

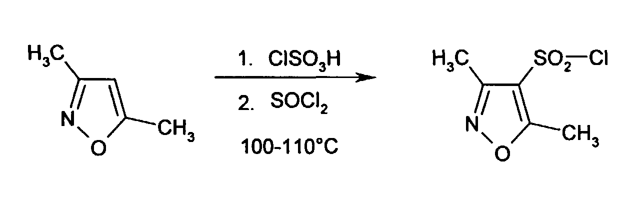

Das bei der Durchführung des erfindungsgemäßen Verfahrens als Ausgangsstoff benötigte 3,5-Dimethylisoxazol der Formel (II) ist bekannt (vgl. J. Het. Chem. 18 (1981), 997-1006).

Die als Reaktionskomponenten benötigten Stoffe Chlorsulfonsäure und Thionylchlorid sind ebenfalls bekannt.

Bei der Durchführung des erfindungsgemäßen Verfahrens arbeitet man im allgemeinen unter Atmosphärendruck. Es ist aber auch möglich, unter erhöhtem Druck zu arbeiten.

Bei der Durchführung des erfindungsgemäßen Verfahrens setzt man auf 1 mol an 3,5- Dimethylisoxazol der Formel (II) im allgemeinen 2 bis 10 mol Chlorsulfonsäure und 1 bis 5 mol Thionylchlorid, vorzugsweise 3 bis 6 mol Chlorsulfonsäure und 1,5 bis 3 mol Thionylchlorid ein.

Im einzelnen verfährt man in der Weise, daß man 3,5- Dimethylisoxazol mit Chlorsulfonsäure umsetzt und nach 1- bis 3-stündiger Reaktionsdauer zusätzlich Thionylchlorid hinzufügt. Die anschließende Aufarbeitung erfolgt in üblicher Weise. Im allgemeinen geht man so vor, daß man das Reaktionsgemisch auf ein Gemisch aus Eis und Wasser gibt und das dabei anfallende Festprodukt absaugt und trocknet.

Das nach dem erfindungsgemäßen Verfahren herstellbare 3,5-Dimethylisoxazol-4-sulfochlorid der Formel (I) ist als wertvolles Zwischenprodukt zur Synthese von Wirkstoffen mit mikrobiziden Eigenschaften bekannt (vgl. WO-A 97-06 171).

So lassen sich fungizid wirksame Benzimidazol-Derivate der Formel 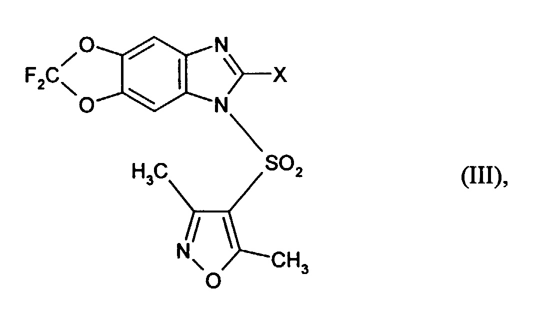 in welcher
- X: für Chlor oder Brom steht,
herstellen, indem man Benzimidazole der Formel 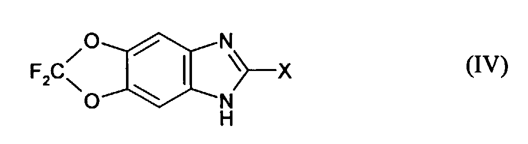 in welcher
- X: die oben angegebene Bedeutung hat
mit 3,5-Dimethylisoxazol-4-sulfochlorid der Formel 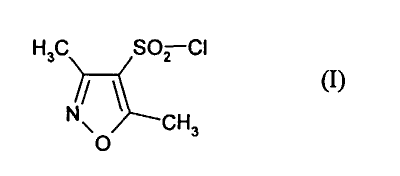 in Gegenwart eines Säurebindemittels, wie Natriumhydrid oder Kaliumcarbonat, und in Gegenwart eines Verdünnungsmittels, wie Tetrahydrofuran oder Acetonitril, umsetzt.

Die Durchführung des erfindungsgemäßen Verfahrens wird durch die folgenden Beispiele veranschaulicht.

### Herstellungsbeispiele

### Beispiel 1

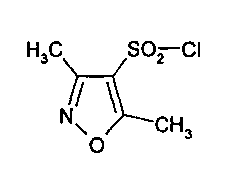

266 ml (4,0 mol) Chlorsulfonsäure werden innerhalb von 45 Minuten unter Rühren in 97,2 g (1 mol) auf 80°C vorgewärmtes 3,5-Dimethylisoxazol eingetropft. Nach beendeter Zugabe wird das Reaktionsgemisch noch 2 Stunden bei 110°C nachgerührt. Anschließend kühlt man auf 60°C ab und tropft innerhalb von 30 Minuten 142,8 g (1,2 mol) Thionylchlorid hinzu. Das Reaktionsgemisch wird noch 1,5 Stunden unter allmählichem Erwärmen bis auf 110°C nachgerührt und dann innerhalb von 45 Minuten unter Rühren in ein Gemisch aus 1,5 kg Eis und 1 Liter Wasser eingetropft. Das entstehende Gemisch wird 30 Minuten gerührt. Danach wird der angefallene Feststoff abgesaugt, mit 1 Liter Wasser gewaschen und getrocknet. Man erhält auf diese Weise 159,8 g (81,7 % der Theorie) an 3,5-Dimethylisoxazol-4-sulfochlorid in Form einer Festsubstanz vom Schmelzpnkt 38,3°C.

### Vergleichsbeispiel A

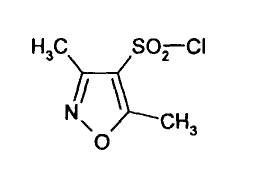

790 g (6,64 mol) Chlorsulfonsäure werden innerhalb von 90 min unter Rühren in 161,4 g (1,66 mol) 3,5-Dimethylisoxazol beginnend bei Raumtemperatur eingetropft, wobei sich das Reaktionsgemisch bis auf 90°C erwärmt. Nach beendeter Zugabe wird das Reaktionsgemisch innerhalb von 20 min auf 100°C erwärmt, dann 1,5 Stunden bei 100°C und danach noch 1,5 Stunden bei 110°C gerührt. Das erhaltene Gemisch wird auf 2,6 kg Eis gegossen. Nach 16stündigem Stehen bei Raumtemperatur wird der anfallende Niederschlag abgesaugt und getrocknet. Man extrahiert das Produkt mit 200 ml heißem Petrolether, filtriert und kocht den Rückstand noch einmal mit 50 mlö Petrolether aus. Die vereinigten Petrolether-Phasen werden 5 Stunden auf 0 bis 5°C gekühlt. Der entstandene Niederschlag wird abgesaugt und getrocknet. Man erhält auf diese Weise 45,7 g (13,8 % der Theorie) an 3,5-Dimethylisoxazol-4-sulfochlorid in Form einer Festsubstanz vom Schmelzpunkt 38-39°C.

### Verwendungsbeispiel I

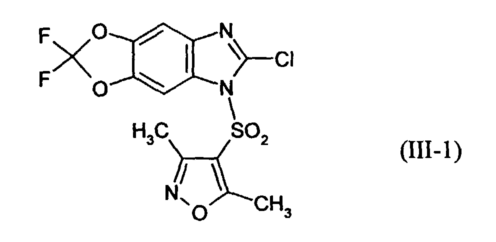

Ein Gemisch aus 96,6 g (0,4 Mol) 2-Chlor-6,6-difluor-[1,3]dioxolo[4,5-f]benzimidazol und 600 ml Acetonitril wird bei Raumtemperatur unter Rühren mit 81,6 g (0,6 Mol) pulverisiertem Kaliumcarbonat versetzt und 10 Minuten bei Raumtemperatur gerührt. Anschließend fügt man 79,2 g (0,4 Mol) 3,5-Dimethyl-isoxazol-4-sulfochlorid hinzu und rührt weitere 20 Stunden bei Raumtemperatur. Das Reaktionsgemisch wird auf 2 Liter Wasser gegossen. Das entstehende Gemisch wird dreimal mit je 500 ml Methylenchlorid extrahiert. Die vereinigten organischen Phasen werden über Natriumsulfat getrocknet und unter vermindertem Druck eingeengt. Der verbleibende Rückstand wird mit Methylenchlorid als Laufmittel an Kieselgel chromatographiert. Man erhält auf diese Weise 117 g (75 % der Theorie) an 1-(3,5-Dimethyl-isoxazol-4-sulfonyl)-2-chlor-6,6-difluor-[1,3]dioxolo-[4,5-f]-benzimidazol in Form einer farblosen Festsubstanz vom Schmelzpunkt 128 bis 131°C.

### Verwendungsbeispiel II

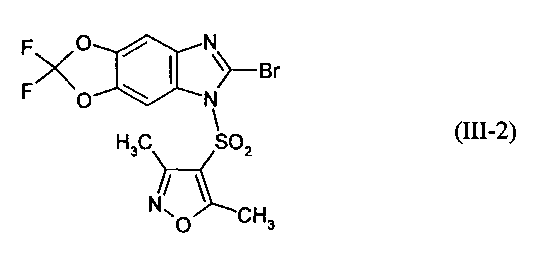

Ein Gemisch aus 1,4 g (5 mmol) 2-Brom-6,6-difluor-[1,3]dioxolo[4,5f]benzimidazol und 30 ml absolutem Tetrahydrofuran wird bei Raumtemperatur unter Rühren mit 0,2 g (5 mmol) Natriumhydrid (60%ig) versetzt und danach 30 Minuten bei Raumtemperatur gerührt. Anschließend fügt man 1,0 g (5,5 mmol) 3,5-Dimethylisoxazol-4-sulfochlorid hinzu und rührt weitere 3 Stunden bei Raumtemperatur. Zur Aufarbeitung wird das Reaktionsgemisch in 100 ml Wasser gegossen. Das entstehende Gemisch wird zweimal mit je 50 ml Essigsäureethylester extrahiert. Die vereinigten organischen Phasen werden über Natriumsulfat getrocknet und unter vermindertem Druck eingeengt. Der verbleibende Rückstand wird mit Methylenchlorid als Laufmittel an Kieselgel chromatographiert. Man erhält auf diese Weise 1,2 g (75% der Theorie) an 1-(3,5-Dimethylisoxazol-4-sulfonyl)-2-brom-6,6-difluor-[1,3]-dioxolo[4,5f]benzimidazol in Form eines farblosen Feststoffes vom Schmelzpunkt 130-134°C.

## Patentansprüche

1. Verfahren zur Herstellung von 3,5-Dimethylisoxazol-4-sulfochlorid der Formel 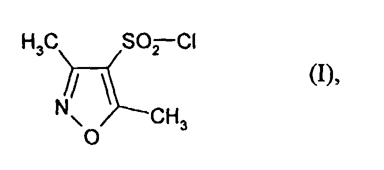 dadurch gekennzeichnet, daß man 3,5-Dimethylisoxazol der Formel 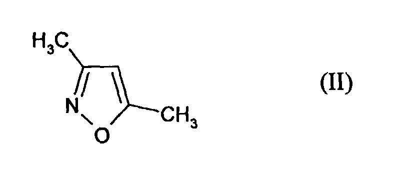 zunächst mit Chlorsulfonsäure und danach zusätzlich mit Thionylchlorid bei Temperaturen zwischen 60°C und 110°C umsetzt.

2. Verfahren gemäß Anspruch 1, dadurch gekennzeichnet, daß man auf 1 mol an 3,5-Dimethylisoxazol der Formel (II) 2 bis 10 mol an Chlorsulfonsäure und 1 bis 5 mol an Thionylchlorid einsetzt.
